(19) Europäisches Patentamt
European Patent Office
Office européen des brevets

(11) Veröffentlichungsnummer: **0 434 174 A1**

(12) EUROPÄISCHE PATENTANMELDUNG

(21) Anmeldenummer: **90250321.8**

(51) Int. Cl.5: **C07D 207/08**

(22) Anmeldetag: **20.12.90**

(30) Priorität: **21.12.89 DD 336093**

(43) Veröffentlichungstag der Anmeldung: **26.06.91 Patentblatt 91/26**

(84) Benannte Vertragsstaaten: **BE CH DE ES FR GB IT LI NL**

(71) Anmelder: **INSTITUT FÜR CHEMISCHE TECHNOLOGIE**
**Rudower Chaussee 5**
**O-1199 Berlin(DE)**

Anmelder: **ZENTRALINSTITUT FÜR ORGANISCHE CHEMIE**
**Rudower Chaussee 5**
**O-1199 Berlin(DE)**

(72) Erfinder: **Ballschuh, Detlef, Dr.**
**Graudenzer Strasse 17**
**O-1034 Berlin(DE)**
Erfinder: **Ohme, Roland, Dr.**
**Waldstrasse 6**
**O-1180 Berlin(DE)**
Erfinder: **Seibt, Horst, Dr.**
**Eugen-Schönhaar-Strasse 15**
**O-1055 Berlin(DE)**
Erfinder: **Gründemann, Egon, Dr.**
**Waldstrasse 4**
**O-1180 Berlin(DE)**

# (54) 3-Methylsulfonylmethyl-4-sulfomethyl-pyrrolidiniumbetaine und Verfahren zu ihrer Herstellung.

(57) Die Erfindung betrifft neue 3-Methylsulfonylmethyl-4-sulfomethyl-pyrrolidinium-betaine (Methylsulfon-sulfobetaine) der allgemeinen Formel I, welche als Zwischenprodukte für weiterführende Synthesen oder, wenn sie einen langkettigen Alkylrest enthalten, als polyfunktionelle Tenside oder Hydrotop eingesetzt werden können. Molare Mengen von Diallylammoniumsalz werden mit Chloressigsäure und der doppeltmolaren Menge Natriumhydrogensulfit in Gegenwart einer katalytischen Menge eines Peroxodisulfats miteinander umgesetzt und die erhaltene Reaktionslösung nach Zugabe einer katalytischen Jodidmenge sowie Calciumcarbonat durch Erwärmen zu 3-Methylsulfonylmethyl-4-sulfomethyl-pyrrolidinium-betainen I umgewandelt, bis die Kohlendioxidentwicklung beendet ist.

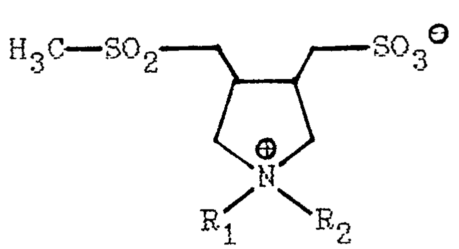

I

EP 0 434 174 A1

# 3-METHYLSULFONYLMETHYL-4-SULFOMETHYL-PYRROLIDINIUM-BETAINE UND VERFAHREN ZU IHRER HERSTELLUNG

Die Erfindung betrifft neue 3-Methylsulfonylmethyl-4-sulfomethyl-pyrrolidinium-betaine (Methylsulfon-sulfobetaine) der allgemeinen Formel I,

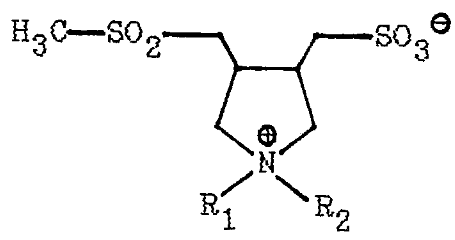

I

worin

$R_1$ und $R_2$ Wasserstoff, einen geradkettigen oder verzweigten Alkylrest mit 1 bis 22 Kohlenstoffatomen oder einen Alkylrest, welcher am Kettenanfang die Gruppe $-CH_2-CONH-$ enthalten kann, darstellen.

Die neuen Methylsulfon-sulfobetaine der Formel I stellen als organische Zwischenprodukte mit Sulfobetaincharakter einen Baustein für weiterführende Synthesen dar. Ist mindestens einer der Substituenten $R_1$ bis $R_2$ ein langkettiger Alkylrest, so können diese Verbindungen als polyfunktionelle Tenside Verwendung finden bzw. kurzkettige Vertreter darüber hinaus als Hydrotope Verwendung finden.

Methylsulfon-sulfobetaine der Formel I sowie Verfahren zu ihrer Herstellung sind bisher nicht bekannt geworden. Andere Dialkylsulfone sind seit langem bekannt [K.Schank in "Methoden der organischen Chemie", Houben-Weyl Bd.E11,S.1145 ff.(1985) bzw. A.Schöberl u. A.Wagner,ebenda Bd.IX, S.232(1955)]. Solche Sulfone werden allgemein durch Umsetzung von Natriumalkylsulfinaten mit Alkylhalogeniden hergestellt. Nach 28 stündigem Erhitzen der Komponenten in Propanol liegen bei den rein aliphatischen Sulfonen [P.Allen et.al., J.org.Chem.16, 767(1951)] die Ausbueten jedoch nicht höher als 27 bis 42 %.

Seit kurzem stehen neuartige sulfobetainsubstituierte Sulfinsäuren bzw. deren Salze als potentielle Ausgangsstoffe für die Synthese von neuen Methylsulfon-sulfobetainen als technisch leicht zugängliche Verbindungen zur Verfügung. Derartige Sulfobetainsulfinate werden durch radikalisch initiierte Sulfocyclosulfinierung von Diallylammoniumsalzen mit Hydrogensulfit nach DD 225 128 A1 bzw. EP 163 319 A3 gewonnen.

Jedoch ergaben Versuche, die nach dem Stand der Technik ausgeführt wurden z.B. mit Natrium-1,1-dimethyl-3-sulfinatomethyl-4-sulfomethyl-pyrrolidinium-betain (Sulfobetainsulfinat) in Wasser/ Alkohol auch nach langen Reaktionszeiten nur unbefriedigende Ausbeuten an Zielprodukten.

Eine andere Methode zur Herstellung von Methylarysulfonen beschreiben A.Courtin, H.-R. von Tobel und G. Auerbach, Helv.Chim. Acta 63, 1412(1980). Methyl-(2-nitrophenyl)-sulfon wird danach durch 24 stündiges Erwärmen von 1 mol 2-Nitrobenzolsulfinsäure, 2 mol Bromessigsäure und 2 mol Natronlauge unter Freisetzung von Kohlendioxid in 91 %iger Ausbeute erhalten. Bei Verwendung von Chloressigsäure anstelle von Bromessigsäure fällt die erzielbare Ausbeute auf 61,5 % ab.

Aber auch die Übertragung dieser Synthesemethode auf oben genanntes Sulfobetain-sulfinat ergab Reaktionslösungen, welche aus einer komplexen Mischung von Ausgangs- und Folgeprodukten bestand, woraus sich das Zielprodukt aufgrund des polaren Charakters von Ausgangs- und Endprodukten nicht abtrennen ließ. Desweiteren sind auch keine Verfahren bekannt geworden, Methylsulfone direkt aus den Ausgangsprodukten von Sulfobetainsulfinaten, den Diallylammoniumsalzen, herzustellen.

Gegenstand der Erfindung ist ein einfaches Syntheseverfahren zur Herstellung von 3-Methylsulfonylmethyl-4-sulfomethyl-pyrollidinium-betainen aus leicht zugänglichen und technisch verfügbaren Ausgangsstoffen, wie Diallylammoniumsalz, Hydrogensulfite und Halogenessigsäuren, das es gestattet, die Verfahrensschritte ohne Bildung von Nebenprodukten bei gleichzeitig hoher Reinheit und Ausbeute des Zielproduktes zu führen.

Erfindungsgemäß werden Diallylammoniumsalze, vorzugsweise Diallylammoniumchloride, der allgemeinen Formel II,

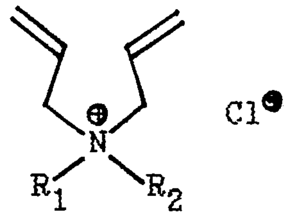

II

in der

$R_1$ und $R_2$ die obengenannten Bedeutungen haben,

mit molaren Mengen einer Halogenessigsäure, vorzugsweise Chloressigsäure und der zweifachmolaren Menge eines Metallhydrogensulfits, vorzugsweise Natriumhydrogensulfit, in Gegenwart einer katalytischen Menge eines Peroxodisulfats miteinander umsetzt und die erhaltene Reaktionslösung nach Zugabe einer katalytischen Menge eines Jodids sowie Calciumcarbonat solange zum Sieden erhitzt bis die Kohlendioxidentwicklung beendet ist. Die Gesamtreaktion, die radikalisch initiierte Sulfocyclosulfinierung des Diallylammoniumsalzes mit gekoppelter Carboxymethylierung/Decarboxylierung des in situ gebildeten Sulfobetainsulfinats veranschaulicht das folgende Schema:

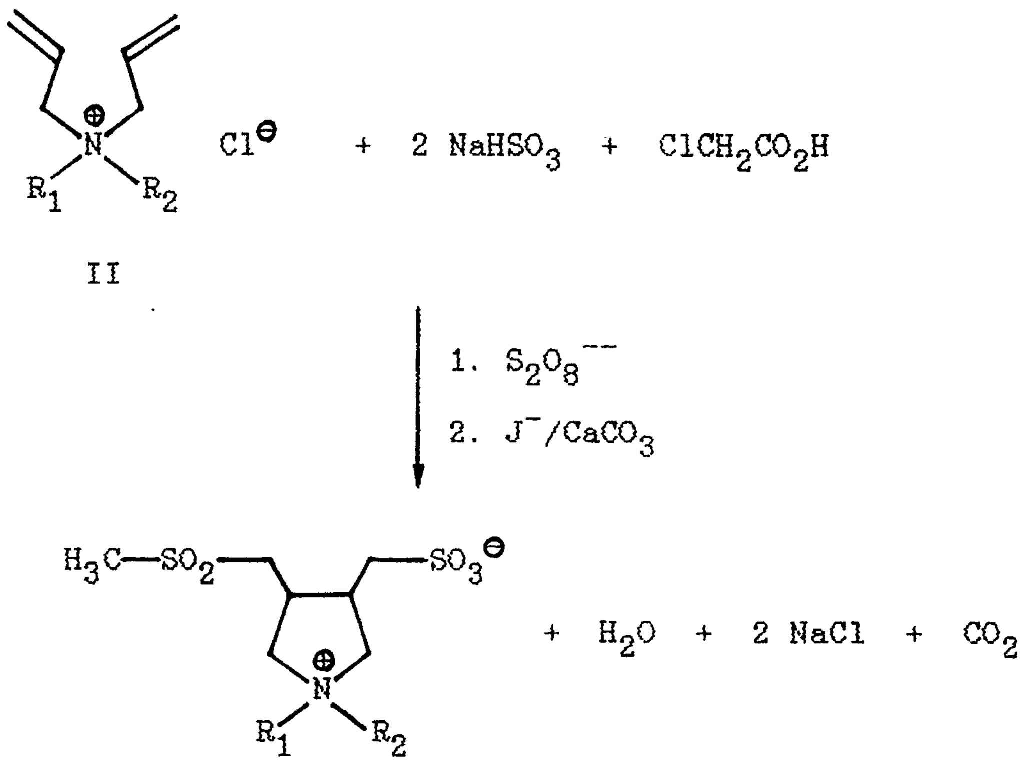


I

Die Sulfocyclosulfinierung eines Diallylammoniumsalzes verläuft nach DD 225 128 A1 nur dann mit maximaler Ausbeute an Sulfocyclosulfinierungsprodukt (Sulfobetainsulfinat), wenn pH-Bedingungen um 2 eingehalten werden. Das Einstellen auf diesen pH-Wert wird durch Zugabe einer Mineralsäure zur Mischung aus Diallylammoniumsalz und Hydrogensulfit erreicht. Entsprechende pH-Wert-Einstellungen sind bisher nicht mit einer Halogenessigsäure vorgenommen worden. Es kann deshalb als ein überraschender Befund angesehen werden, daß beispielsweise der Zusatz von 1 mol Chloressigsäure zu einer Mischung aus 1 mol Diallylammoniumsalz und 2 mol technischer Natriumhydrogensulfitlösung den optimalen Start-pH-Wert um 2 ergibt; also Bedingungen wie sie der obigen Zielstellung entsprechen. Die Reihenfolge des Zusammengebens der Reaktionsteilnehmer ist für den Reaktionsablauf ohne Bedeutung.

In Abhängigkeit von der Qualität der verwendeten Natriumhydrogensulfitlösung können geringe PH-Wert-Schwankungen auftreten. Sie sind dadurch zu korrigieren, daß entweder eine geringe Menge einer Mineralsäure zugesetzt oder die Reaktion mit einer Teilmenge der Halogenessigsäure gestartet und die Restmenge der Halogenessigsäure erst während der Reaktion zugesetz wird.

Bei Einsatz von z.B. tertiären Diallylaminen als Ausgangsprodukte der Umsetzung sollten diese zweckmäßigerweise in der Halogencarbonsäure gelöst und nach Zugabe der Hydrogensulfitlösung der Start-pH-Wert mit einer Mineralsäure eingestellt werden. Da bei tertiären Diallylaminen und einem Start-pH-Wert von 2,5 die Sulfocyclosulfinierung bereits vollständig abläuft, ist weniger als die moläquivalente Menge an Mineralsäure für die Bildung des Diallylammoniumsalzes notwendig.

Zur so vorbereiteten Startlösung, welche eine Temperatur zwischen 20 bis 30 °C haben sollte, wird zur Durchführung des ersten Verfahrensschritts 1 bis 5 Mol-% Peroxodisulfat, bezogen auf das eingesetzte Diallylammoniumsalz, hinzugesetzt, um die Sulfocyclosulfinierungsreaktion auszulösen.

Es hat sich dabei als zweckmäßig erwiesen, das Peroxodisulfat in zwei Portionen hinzuzusetzen, um einen möglichst vollständigen Umsatz zu erreichen, wobei meistens Mengen von 2 mal 2 Mol-%, bezogen auf das eingesetzte Diallylammoniumsalz, völlig ausreichend sind. Als Peroxodisulfate sind Natrium-, Kalium- oder Ammoniumperoxodisulfat geeignet, welche entweder als Lösung oder pulverförmig eingesetzt werden können. Die anfänglich fahlgelbe Startlösung färbt sich nach der Peroxodisulfatzugabe rot bis blutrot, hellt sich aber kurz nach Durchlaufen des Temperaturmaximums deutlich auf.

Zur Einleitung der zweiten Synthesestufe wird zur Neutralisation der sauren Reaktionlösung Calciumcarbonat sowie eine katalytische Menge eines löslichen Jodids, z.B. Natrium-, Kalium- oder Ammoniumjodid, gegebenenfalls Jodessigsäure, hinzugefügt. Bei einer Einsatzmenge von 5 bis 13 mmol Natrium-, Kalium- oder Ammoniumjodid/mol Diallylammoniumsalz, vorzugsweise 7 mmol Natrium-, Kalium-oder Ammoniumjodid/mol Diallylammoniumsalz, werden im Verlaufe von 1 bis 2 Stunden vollständige Umsätze erreicht. Während Mengen unter 3 mmol o.g. Jodide keine katalytische Wirkung mehr erkennen lassen, beschleunigen größere Mengen als 7 mmol die Reaktion weiter; verfärben jedoch später die kristallinen Zielprodukte.

Unter Siedekühlung wird solange weiter erwärmt bis sich das intermidiär bildende Calciumsalz des Carboxy-methylsulfon-sulfobetains unter Decarboxylierung und Rückbildung von Calciumcarbonat zum Methylsulfon-sulfobetain gemäß Formel I zersetzt hat. Der Endpunkt der Reaktion ist leicht am Ausfallen des Calciumcarbonats aus der Reaktionslösung bzw. der Beendigung der Kohlendioxidentwicklung (Blasenzähler) zu erkennen.

Zur Neutralisation der obigen Reaktionslösung können neben Calciumcarbonat auch andere Metallcarbonate, wie Barium- oder Zinkcarbonat, eingesetzt werden, jedoch bieten diese gegenüber Calciumcarbonat keine Vorteile. Ferner können auch anstelle von Calciumcarbonat zur Neutralisation die Hydroxide oder Carbonate der Alkalimetalle oder des Ammoniaks verwendet werden; der Nachteil besteht jedoch darin, daß die dann entstehenden gelösten Carbonate sich nur schwierig vom Zielprodukt abtrennen lassen und andererseits auch der Endpunkt der Umsetzung schwer zu erkennen ist. Währenddessen läßt sich Calciumcarbonat leicht durch Filtration von der Reaktionslösung abtrennen und kann für weitere Ansätze erneut eingesetzt werden.

Wird die Carboxymethylierung des Sulfocyclosulfinierungsprodukts ohne Jodidzusatz ausgeführt, findet zwar in der Anfangsphase die gewünschte Umsetzung statt, sie verlangsamt sich aber mit fortschreitender Reaktionszeit derart, daß auch nach langer Reaktionszeit kein vollständiger Umsatz erreichbar ist. Das hat zur Folge, daß sich das Zielprodukt aufgrund des polaren Charakters von Ausgangs-, Zwischen- und Endprodukt nicht abtrennen läßt.

Besonders reine, d.h. elektrolytfreie Methylsulfon-sulfobetaine werden nach einer weiteren Ausführungsform des erfindungsgemäßen Verfahrens erhalten, wenn zunächst das Sulfocyclosulfinierungsprodukt des Diallylammoniumsalzes mit Chloressigsäure in Gegenwart von Jodid zum Zwischenprodukt, Carboxymethylsulfon-sulfobetain, umgesetzt wird und dieses nach Isolierung und ggf. zusätzlicher Umkristallisation mit Calciumcarbonat zum Methylsulfonsulfobetain gemäß Formel I decarboxyliert.

In einer weiteren Ausführungsform des Verfahrens können auch vorher isolierte Sulfobetainsulfinsäuren mit Chloressigsäure, katalytischen Jodidmengen und Calciumcarbonat zu den Zielverbindungen umgesetzt werden. Jedoch bietet dieser Syntheseweg keinen prinzipiellen Verfahrensvorteil, da die dafür notwendigen Ausgangs-sulfobetain-sulfinsäuren zuvor aus den entsprechenden Diallylammoniumsalzen hergestellt werden müssen.

Zusammenfassend besteht der Vorteil der Erfindung in einer einfachen Synthese, welche ohne die Isolierung von Zwischenprodukten, die bisher unbekannten 3-Methylsulfonylmethyl-4-sulfomethylpyrrolidinium-betaine der allgemeinen Formel I aus Diallylammoniumsalzen direkt zugänglich macht. Als Ausgangsstoffe werden im technischen Maßstab verfügbare Zwischenprodukte genutzt, die mit einfachen Mitteln in kurzen Reaktionszeiten zur Umsetzung gebracht werden können.

Die Erfindung soll durch die nachfolgenden Beispiele näher erläutert werden.

Beispiel 1

1,1-Dimethyl-3-methylsulfonylmethyl-4-sulfomethyl-pyrrolidinium-betain ($R_1 = R_2 = CH_3$ in der allgemeinen Formel I)

a) In einem 1,5 1 Sulfierkolben, der mit einem Rührer, Thermometer, Rückflußkühler sowie einer Heizquelle ausgerüstet ist, werden 269,5 g (1 mol) 60 %ige wäßrige Dimethyldiallyl-ammoniumchloridlösung, 118,1 g (1 mol) 80 %ige wäßrige Chloressigsäure sowie 539 g (2,02 mol) 39 %ige technische Natriumhydrogensulfitlösung vorgelegt und miteinander vermischt. Man erhält eine fahlgelbe, homogene Lösung von 21 °C und einem Start-pH-Wert von 2,02. (pH-Wert-Schwankungen, d. h. Abweichungen vom optimalen pH-Wert 2, sind bei verschiedenen Ansätzen hauptsächlich von der Qualität der eingesetzten technischen Natriumhydrogensulfitlösung abhängig und können entweder durch Zusatz geringer Mengen von Mineralsäure oder durch Weglassen eines Teils der Chloressigsäure, welche dann erst nach erfolgter Umsetzung zugesetzt wird, korrigiert werden). Zur Initiierung der Umsetzung fügt man nun zunächst 4,76 g (2 Mol-%) Natriumperoxodisulfat und eine Minute später - die Reaktionslösung hat sich inzwischen blutrot gefärbt und die Temperatur ist auf 60 °C gestiegen - nochmals 2 Mol-% Natriumperoxodisulfat hinzu. Bereits nach einer weiteren Minute wird das Reaktionstemperaturmaximum von 76 °C erreicht. Man fügt zur Reaktionslösung 1,05 g (7 mmol) Natriumjodid sowie 50 g (0,5 mol) Calciumcarbonat hinzu. Nachdem sich die Hauptmenge des Calciumcarbonates unter Kohlendioxidentwicklung gelöst hat, setzt man auf dem Rückflußkühler einen Blasenzähler auf und erhitzt für 10 bis 12 Stunden zum Sieden auf 110 °C bis die Kohlendioxidentwicklung praktisch beendet ist.

(Man kann aber auch zunächst auf den Zusatz von Calciumcarbonat verzichten und dafür die Reaktionslösung für 90 Minuten zum Sieden erhitzen und das Zwischenprodukt, 1,1-Dimethyl-3-carboxymethylsulfonylmethyl-4-sulfomethyl-pyrrolidinium-betain,

als kristallines Dihydrat aus der abgekühlten Lösung isolieren und dieses erst danach mit Calciumcarbonat zur Umsetzung bringen - vgl. Beispiel 1b).

Man läßt etwas abkühlen, filtriert vom Calciumcarbonat ab und bewahrt das Filtrat über Nacht zur Kristallisation im Kühlschrank auf. Zur Aufarbeitung saugt man den Kristallbrei scharf von der Mutterlauge ab, wäscht die Kristalle mit wenig Wasser und zweimal mit Alkohol und erhält nach dem Trocknen an der Luft ein farbloses Kristallpulver, welches zur Feinreinigung noch aus Wasser umkristallisiert werden kann (F:ab 270 °C unter Zersetzung). Aus den vereinigten Mutter- und Waschlaugen kann nach weiterem Einengen sowie Stehenlassen noch eine kristalline Fraktion des Zielproduktes gewonnen werden $^{13}$C-NMR-Spektrum ($D_2O$, externer Standard Tetramethylsilan; TMS; $\delta = 0{,}0$ ppm): Die Zahlenangaben an den Atomsymbolen entsprechen den chemischen Verschiebungen für die cis-Konfiguration (3,4-Position) in ppm.

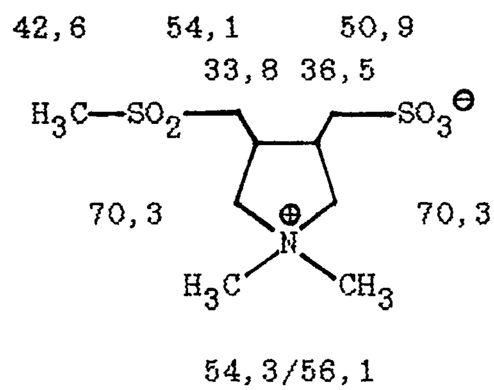


Die $N\text{-}CH_3$-Gruppen sind nicht äquivalent und wie bei den $N\text{-}CH_2$-Gruppen erfolgt noch zusätzlich Signalaufspaltung durch das $^{14}$-Quadrupolmoment.

b) 36,54 g (0,1 mol) 1,1-Dimethyl-3-carboxymethylsulfonylmethyl-4-sulfomethyl-pyrrolidinium-betain-dihydrat (nach Beispiel 1a) hergestelltes sowie isoliertes und aus Wasser umkristallisiertes Zwischenprodukt; F: ab 290 °C unter Zersetzung), 5 g (0,05 mol) Calciumcarbonat und 60 g Wasser werden 8 Stunden unter Freisetzung von Kohlendioxid zum Sieden erhitzt und vom ausgefallenen Calciumcarbonat abgetrennt. Aus dem etwas eingeengten Filtrat kristallisiert das Methylsulfon-sulfobetain in praktisch

quantitativer Ausbeute als reine Verbindung. Das $^{13}C$-NMR-Spektrum ist mit dem nach Beispiel 1a) hergestellten Produkt identisch.

c) 30,7 g (0,1 mol) 1,1-Dimethyl-3-sulfinsäuremethyl-4-sulfomethyl-pyrrolidinium-betain-dihydrat (hergestellt aus Dimethyl-diallylammoniumchlorid und Natriumhydrogensulfit; vgl. DD 225 128 Beispiel 13), 11,8 g (0,1 mol) 80 %ige wäßrige Chloressigsäure, 10 g (0,1 mol) Calciumcarbonat, 0,15 g (1 mmol) Natriumjodid sowie 50 g Wasser werden miteinander gemischt und bis zur Beendigung der Kohlendioxidentwicklung (8 Stunden) zum Sieden erhitzt. Man filtriert vom ausgefallenen Calciumcarbonat ab und arbeitet nach Beispiel 1a auf. Man erhält ein kristallines Präparat, welches mit denen nach den Beispielen 1a und 1b hergestellten identisch ist.

Beispiel 2

1-Dodecyl-1-methyl-3-methylsulfonylmethyl-4-sulfomethyl-pyrrolidinium-betain
($R_1 = CH_3$; $R_2 = C_{12}H_{25}$ in der allgemeinen Formel I)

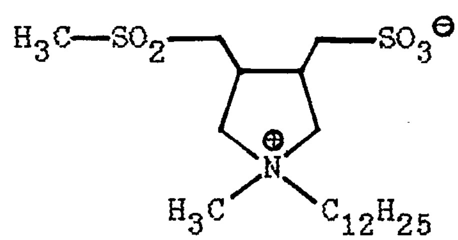

Man verfährt nach Beispiel 1a und setzt eine Mischung von pH 2 aus 36 g (0,1 mol) Dodecyl-methyl-diallylammoniumbromid, 11,8 g (0,1 mol) 80 %ige wäßrige Chloressigsäure, 53,9 g (0,202 mol) 39 %ige technische Natriumhydrogensulfitlösung mit zwei Portionen von je 0,46 g (2 mol-%) Ammoniumperoxodisulfat um. Man fügt zur Reaktionslösung 5 g (0,05 mol) Calciumcarbonat und 0,15 g (1 mmol) Natriumjodid hinzu und erwärmt bis zur Beendigung der Kohlendioxidentwicklung zum Sieden. Die erhaltene Reaktionslösung kann zur Isolierung des reinen Sulfobetain-methylsulfons weiter eingeengt und mit Ethanol extrahiert werden. Wird eine Probe des Reaktionsproduktes mit Wasser verdünnt, erhält man eine stark schäumende Tensidlösung.

**Ansprüche**

1. 3-Methylsulfonylmethyl-4-sulfomethyl-pyrrolidinium-betaine der allgemeinen Formel I,

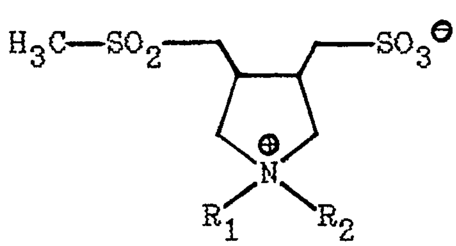

I

worin
$R_1$ und $R_2$ Wasserstoff, einen geradkettigen oder verzweigten Alkylrest mit 1 bis 22 Kohlenstoffatomen oder einen Alkylrest, welcher am Kettenanfang die Gruppe $-CH_2-CONH-$ enthalten kann, darstellen.

2. Verbindungen nach Anspruch 1, worin $R_1$ und $R_2$ $-CH_3$ bedeuten.

3. Verbindungen nach Anspruch 1, worin $R_1$ $-CH_3$ und $R_2$ $-C_{12}H_{25}$ bedeuten.

4. Verfahren zur Herstellung von 3-Methylsulfonylmethyl-4-sulfomethyl-pyrrolidinium-betainen der allgemeinen Formel I nach Anspruch 1 bis 3, wobei Diallylammoniumsalze, vorzugsweise Diallylammoniumchloride, der allgemeinen Formel II,

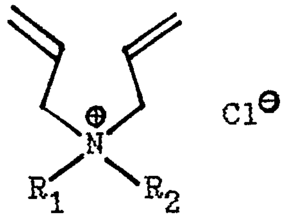

II

in der
$R_1$ und $R_2$ die obengenannten Bedeutungen haben,
mit molaren Mengen einer Halogenessigsäure, vorzugsweise Chloressigsäure und der zweifachmolaren Menge eines Metallhydrogensulfits, vorzugsweise Natriumhydrogensulfit, in Gegenwart einer katalytischen Menge eines Peroxodisulfats miteinander umgesetzt und die erhaltene Reaktionslösung nach Zugabe einer katalytischen Menge eines Jodids sowie Calciumcarbonat solange zum Sieden erhitzt wird bis die Kohlendioxidentwicklung beendet ist.

**5.** Verfahren nach Anspruch 4, gekennzeichnet dadurch, daß im ersten Verfahrensschritt 1 bis 5 Mol-% Peroxodisulfat, bezogen auf das eingesetzte Diallylammoniumsalz, als Katalysator zugesetzt werden.

**6.** Verfahren nach Anspruch 4 und 5, gekennzeichnet dadurch, daß das Peroxodisulfat in zwei Portionen von jeweils 2 Mol-%, bezogen auf das eingesetzte Diallylammoniumsalz, zugesetzt wird.

**7.** Verfahren nach Anspruch 4, gekennzeichnet dadurch, daß im zweiten Verfahrensschritt 5 bis 13 mmol Natrium-, Kalium- oder Ammoniumjodid/mol Diallylammoniumsalz als Katalysator zugesetzt werden.

**8.** Verfahren nach Anspruch 4 und 7, gekennzeichnet dadurch, daß 7 mmol Natrium-, Kalium- oder Ammoniumjodid/mol Diallylammoniumsalz zugesetzt wird.

Europäisches Patentamt

# EUROPÄISCHER RECHERCHENBERICHT

Nummer der Anmeldung

**EP 90 25 0321**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,A | GB-A-2 080 293 (AKADEMIE DER WISSENSCHAFTEN) * Insgesamt * --- | 1-8 | C 07 D 207/08 |
| A | US-A-4 528 383 (SCHMITT) * Insgesamt * --- | 1-8 | |
| A,D | EP-A-0 163 319 (AKADEMIE D. WISSENSCHAFTEN) * Insgesamt * ----- | 1-8 | |

| RECHERCHIERTE SACHGEBIETE (Int. Cl.5) |
|---|
| C 07 D 207/00 |

Der vorliegende Recherchenbericht wurde für alle Patentansprüche erstellt

| Recherchenort | Abschlußdatum der Recherche | Prüfer |
|---|---|---|
| Den Haag | 15 März 91 | KISSLER B.E. |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument